# FASCICULE DE BREVET EUROPEEN

(11) **EP 3 645 701 B1**
(45) Date de publication et mention de la délivrance du brevet: **01.12.2021**
(21) Numéro de dépôt: 18749002.4
(22) Date de dépôt: 26.06.2018
(51) Int. Cl.: C12M 1/22, C12M 1/12, C12M 1/36, B29C 39/02

(54) **INSTALLATION DE PREPARATION D'UN SUPPORT DE CULTURE**
ANLAGE ZUR ZUBEREITUNG EINES KULTURTRÄGERS
FACILITY FOR PREPARING A CULTURE SUPPORT

(30) Priorité: 29.06.2017 FR 1756045
(43) Date de publication de la demande: 06.05.2020
(73) Titulaire: Intelligence Artificielle Applications, 34060 Montpellier Cedex 2 (FR)
(72) Inventeur: CUREL, Christian, 34880 Laverune (FR); ROCH, Michel, 34670 Saint Bres (FR)
(74) Mandataire: Ipsilon
(86) Numéro de dépôt international: PCT/FR2018/051546
(87) Numéro de publication internationale: WO 2019/002745

(56) Documents cités:
- EP-A2- 0 447 893
- WO-A1-2017/089911
- US-A- 5 020 297

## Description

### DOMAINE DE L'INVENTION

L'invention concerne une installation de préparation d'un support de culture.

Elle concerne plus particulièrement une installation de préparation d'un support de culture, tel qu'une boîte de Pétri, ladite installation comprenant un poste de coulée, au moins une surface, telle qu'un plateau, de réception du support de culture, et un ou plusieurs contenants de produit à couler à l'intérieur dudit support de culture.

### ART ANTÉRIEUR

Les milieux de culture sont largement utilisés en microbiologie, pour la culture de microorganismes. Les milieux de culture peuvent se distinguer par leur composition, mais ils se distinguent également par leur consistance. On distingue ainsi les milieux de culture solides ou semi-solides et les milieux liquides, encore appelés bouillons.

Les milieux de culture solides ou semi-solides comprennent un agent gélifiant en une teneur au moins égale à 5 grammes par litre, de préférence au moins égale à 10 grammes par litre et sont généralement obtenus par ajout de cet agent gélifiant à un milieu liquide. Cet agent gélifiant est généralement de l'agar-agar ou gélose. Il s'agit d'un poly galactoside.

Jusqu'à présent, de tels milieux solides ou semi-solides sont stockés en flacon en verre stérile. Chaque flacon est généralement d'une contenance voisine de 200 ml. Le milieu contenu dans ledit flacon doit, pour être utilisable, être fondu. Le flacon est donc placé pendant 60 minutes dans un bain-marie thermostaté à 95°C. Le milieu en surfusion est refroidi dans un bain d'eau thermostaté à une température généralement comprise entre 44 et 47°C pour un maintien à l'état liquide dudit milieu et éviter une cuisson des peptones contenues dans ledit milieu.

Pour la préparation d'un support de culture, tel qu'une boîte de Pétri, ensemencé en surface, on prélève un flacon du bain d'eau thermostaté, on le place dans un environnement stérile dans lequel la boîte de Pétri est présente. On ouvre le flacon et on déverse au moins une partie de son contenu dans la boîte de Pétri.

Pour la préparation d'un ensemencement dit dans la masse d'un support de culture, tel qu'une boîte de Pétri, on dépose l'échantillon dans ladite boîte vide puis on prélève un flacon du bain d'eau thermostaté, on le place dans un environnement stérile dans lequel la boîte de Pétri est présente. On ouvre le flacon et on déverse au moins une partie de son contenu dans la boîte de Pétri.

La préparation d'un tel support de culture s'avère longue, fastidieuse, et nécessite de nombreux flacons lorsque la quantité de support à préparer est importante, entraînant des contraintes de stockage et rendant l'automatisation difficile.

En outre, comme un tel milieu de culture ne peut plus être conservé une fois placé en surfusion pendant plusieurs heures, le flacon qui le contient est de faible volume, pour réduire le risque de perte de matière, ce qui accroît les difficultés de stockage.

Enfin, la vidange automatique d'un tel flacon rend nécessaire la présence d'une prise d'air, entraînant des difficultés de manipulation du flacon dans des conditions stériles.

Il est apparu très récemment un conditionnement en poche de milieu de culture à reconstituer comme l'illustre la demande WO2017/089911. Toutefois une installation permettant l'utilisation d'une telle poche n'est pas décrite dans ce document. US5020297A divulgue une installation de préparation d'un support de culture, tel qu'une boîte de Pétri, ladite installation comprenant un poste de coulée, une surface telle qu'un plateau de réception du support de culture et une buse d'addition du milieu de produit à couler contenant un gélifiant.

Il est également connu des installations de coulage de milieu de culture dans lesquelles le milieu de culture est stocké à l'extérieur de l'installation dans un autoclave. Une telle installation permet l'utilisation d'un seul milieu à la fois, ce milieu devant être préparé en quantité importante.

### BUT ET RÉSUMÉ

Un but de l'invention est de proposer une installation du type précité dont les conceptions permettent une simplification de la préparation du support de culture, un risque de perte de matière réduit et une plus grande souplesse d'utilisation desdits contenants.

À cet effet, l'invention a pour objet une installation de préparation d'un support de culture, tel qu'une boîte de Pétri, ladite installation comprenant un poste de coulée, au moins une surface, telle qu'un plateau de réception du support de culture et un ou plusieurs contenants de produit à couler à l'intérieur dudit support de culture, caractérisée en ce que le ou au moins l'un des contenants de produit à couler, appelé contenant de premier type, est une poche souple présentant un orifice de vidange et contenant un milieu de culture solide ou semi-solide comprenant un gélifiant, en ce que le poste de coulée comprend une enceinte équipée de moyens de chauffage aptes à chauffer l'air intérieur de l'enceinte, en ce que ladite enceinte est configurée pour loger au moins deux contenants de premier type, en ce que l'enceinte de stockage du ou des contenants de premier type du poste de coulée et la surface de réception du support de culture sont déplaçables l'une par rapport à l'autre et en ce que l'installation comprend au moins une configuration dans laquelle la surface de réception du support de culture s'étend au-dessous et à l'aplomb de l'enceinte de stockage du ou des contenants de premier type.

Le stockage du milieu de culture solide ou semi-solide en poche souple, c'est-à-dire déformable sous l'effet d'une pression exercée sur ladite poche à l'état liquide du milieu de culture, permet de moduler plus aisément le volume de la poche et facilite le stockage de ladite poche.

En outre, une telle poche est apte à être maintenue à une température correspondant à la température de surfusion du milieu de culture par simple chauffage de l'air ambiant à l'intérieur duquel ladite poche est placée. Il en résulte un chauffage par conduction de ladite poche au contact de l'air chaud.

Selon un mode de réalisation de l'invention, les moyens de chauffage sont configurés pour maintenir l'intérieur de l'enceinte de stockage du ou des contenants de premier type du poste de coulée à une température comprise entre 44 et 47°C.

Selon un mode de réalisation de l'invention, le poste de coulée et la surface de réception du support de culture sont logés à l'intérieur d'une enceinte équipée de moyens de production d'un flux d'air, de préférence laminaire, et de filtration dudit flux d'air ou à l'intérieur d'une salle blanche. Il est ainsi possible de procéder aux opérations de préparation du support de culture dans des conditions stériles.

Selon un mode de réalisation de l'invention, le poste de coulée comprend des moyens de vidange des contenants de produit à couler, lesdits moyens de vidange comprenant des moyens de pompage des produits à couler avec au moins une partie des moyens de pompage disposée à l'intérieur de l'enceinte de stockage du ou des contenants de premier type, cette partie des moyens de pompage disposée à l'intérieur de l'enceinte de stockage du ou des contenants de premier type comprenant de préférence au moins une pompe telle qu'une pompe péristaltique par contenant de premier type. Ainsi, le contenant de produit à couler est équipé d'un orifice de vidange raccordable à une buse par un conduit tubulaire, et la pompe péristaltique est équipée d'une tête de pompe rotative au contact de laquelle le conduit tubulaire est apte à être positionné, pour une vidange automatique du contenant, en fonction de la rotation de la tête de pompe.

Selon un mode de réalisation de l'invention, l'installation comprend une unité de pilotage des moyens de pompage, des moyens de saisie et/ou d'enregistrement de données relatives à la composition du support de culture à préparer et les moyens de pompage sont configurés pour être pilotés par l'unité de pilotage en fonction desdites données.

Selon un mode de réalisation de l'invention dans laquelle l'installation comprend plusieurs contenants de produit à couler à savoir au moins un contenant de produit à couler de premier type et au moins un contenant de produit à couler dit de deuxième type, le ou au moins l'un des contenants de deuxième type se présentant sous forme d'une poche contenant un liquide, le poste de coulée comprend, jouxtant l'enceinte de stockage du ou des contenants de premier type, et isolé thermiquement de ladite enceinte, un compartiment de stockage du ou des contenants de deuxième type. A l'intérieur de ce compartiment, les contenants de deuxième type peuvent être conservés à température ambiante ou à une autre température. Il en résulte la possibilité de procéder à la préparation de support de culture contenant uniquement un milieu de culture solide ou semi-solide, ou à la préparation de support de culture contenant à la fois un milieu de culture solide ou semi-solide et un liquide, tel qu'un additif thermosensible, un antibiotique ou autre.

Selon un mode de réalisation de l'invention dans laquelle le ou chaque contenant de produit à couler est équipé d'un orifice de vidange raccordable à une buse, le poste de coulée est équipé d'un porte-buse pour contenant de premier type et éventuellement d'un porte-buse pour contenant de deuxième type lorsque le ou les contenants de deuxième type sont présents, et chaque porte-buse est configuré pour délimiter au moins un logement traversant servant à la réception d'une buse.

Selon un mode de réalisation de l'invention, au moins un logement traversant, apte à recevoir une buse, du porte-buse pour contenant de premier type, et au moins un logement traversant, apte à recevoir une buse, du porte-buse pour contenant de deuxième type présentent au moins une configuration dans laquelle ils sont positionnables à l'aplomb de la surface de réception du support de culture pour permettre en parallèle un coulage simultané du contenu des contenants de premier type et du contenu des contenants de deuxième type.

Selon un mode de réalisation de l'invention, chaque logement traversant est muni de moyens d'indexation en position de la buse à l'intérieur dudit logement. Il en résulte un positionnement précis de la buse, pour éviter tout contact de l'extrémité libre de la buse avec le contenu du support de culture.

Selon un mode de réalisation de l'invention, chaque logement traversant est muni de moyens de détection de la présence de la buse à l'intérieur dudit logement.

Selon un mode de réalisation de l'invention, au moins un logement traversant, apte à recevoir une buse, du porte-buse pour contenant de premier type, et au moins un logement traversant, apte à recevoir une buse, du porte-buse pour contenant de deuxième type présentent au moins une configuration dans laquelle ils sont positionnables à l'aplomb de la surface de réception du support de culture pour permettre en parallèle un coulage simultané du contenu des contenants de premier type et du contenu des contenants de deuxième type.

Selon un mode de réalisation de l'invention, chaque logement traversant de porte-buse apte à recevoir une buse est monté orientable autour d'un axe dit horizontal parallèle au plan d'appui au sol de l'installation ou au plan support formé par la surface de réception du support de culture.

Cette possibilité d'orientation du porte-buse et des buses associées permet notamment d'éviter la production d'éclaboussures lors du coulage. Elle facilite également le chargement des buses en position verticale. Enfin, elle permet plus facilement de couler simultanément le contenu des contenants de premier type et le contenu des contenants de deuxième type.

Selon un mode de réalisation de l'invention, le porte-buse pour contenant de premier type est logé au moins partiellement à l'intérieur de l'enceinte de stockage de contenants de premier type, chaque buse de contenant de premier type débouche à l'extérieur de l'enceinte de stockage des contenants de premier type à l'état inséré de la buse dans un logement traversant du porte-buse pour contenants de premier type, et chaque logement traversant du porte-buse apte à recevoir une buse est équipé de moyens de chauffage s'étendant au moins partiellement autour dudit logement. Il en résulte un risque réduit de prise en masse du milieu de culture à l'intérieur de la buse.

Selon un mode de réalisation de l'invention, l'enceinte de stockage de contenants de premier type comprend une face du dessus, une face du dessous et des faces latérales et le porte-buse pour contenant de premier type débouche, à l'extérieur de l'enceinte, via la face du dessous de l'enceinte au niveau d'une partie de la face du dessous de l'enceinte formée par une paroi souple, telle qu'une membrane.

Selon un mode de réalisation de l'invention, le porte-buse pour contenant de deuxième type est porté par un bras pivotant, monté mobile à pivotement autour d'un axe orthogonal au plan support formé par la surface de réception du support de culture pour le passage du porte-buse d'une position dite active, dans laquelle il s'étend au-dessous et à l'aplomb de l'enceinte de stockage du ou des contenants de premier type dans l'espace laissé libre entre l'enceinte de stockage et le plan support formé par la surface de réception du support de culture, à une position inactive dans laquelle il s'étend à l'extérieur dudit espace.

Selon un mode de réalisation de l'invention, l'enceinte de stockage de chaque contenant de premier type est équipée de moyens de maintien des contenants de premier type à l'état suspendu à l'intérieur de ladite enceinte, lesdits moyens de maintien comprenant, pour chaque contenant de premier type à stocker, un boîtier grillagé à l'intérieur duquel ledit contenant de premier type est apte à être logé, ledit boîtier étant équipé d'au moins un organe de maintien à l'état suspendu à l'intérieur de ladite enceinte, ledit orifice de vidange du contenant de premier type s'étendant, à l'état logé du contenant de premier type dans le boîtier et à l'état suspendu du boîtier, en partie inférieure du boîtier. Il en résulte une simplicité de rechargement en contenant de l'installation et une facilité de nettoyage.

Selon un mode de réalisation de l'invention, l'enceinte de stockage des contenants de premier type est équipée de moyens d'agitation de l'air contenu dans ladite enceinte. Ces moyens d'agitation de l'air de l'enceinte permettent une homogénéisation de la chaleur à l'intérieur de l'enceinte.

Selon un mode de réalisation de l'invention, le poste de coulée se présente sous forme d'un bloc parallélépipédique comprenant une partie fixe et une partie mobile, ladite partie mobile comprenant au moins la face du dessous et l'une des faces latérales dudit bloc, cette partie mobile étant déplaçable à coulissement par rapport à la partie fixe à la manière d'un tiroir entre une position écartée de la partie fixe et une position rapprochée de la partie fixe pour permettre, en position écartée, l'accès à l'intérieur du volume formé par le bloc. Grâce à cette conception, il en résulte un rechargement aisé en contenant de l'installation.

Selon un mode de réalisation de l'invention, l'installation comprend un poste d'ensemencement, en ce que le poste d'ensemencement et la surface de réception du support de culture sont déplaçables l'un par rapport à l'autre et en ce que l'installation comprend au moins une configuration dans laquelle la surface de réception du support de culture s'étend au-dessous et à l'aplomb du poste d'ensemencement.

### BRÈVE DESCRIPTION DES FIGURES

L'invention sera bien comprise à la lecture de la description suivante d'exemples de réalisation, en référence aux dessins annexés dans lesquels :
- La figure 1 représente une vue schématique en perspective d'une installation conforme à l'invention, en configuration d'utilisation du poste de coulée.
- La figure 2 représente une vue schématique en perspective d'une installation conforme à l'invention, en configuration de chargement du poste de coulée.
- La figure 3 représente une vue schématique en perspective prise de dessus, du poste de coulée, en position de chargement du poste de coulée et déployée des bras.
- La figure 4 représente une vue de la partie mobile du bloc constitutif du poste de coulée, avec une vue de détail du bras porte-buse et du bras anti-goutte.
- La figure 5 représente une vue schématique partielle illustrant le trajet du milieu de culture entre la poche et le support de culture, en position inclinée des buses.
- La figure 6 représente une vue schématique illustrant le porte-buse pour contenant de premier type en position non inclinée.
- La figure 7 représente deux vues d'une poche, l'une au cours de son introduction dans un boîtier grillagé, l'autre à l'état introduit dans ledit boîtier.

### DESCRIPTION DÉTAILLÉE

Comme mentionné ci-dessus, l'installation 1 objet de l'invention est destinée à permettre la préparation d'un support 32 de culture se présentant ici sous forme d'une boîte de Pétri, par coulage de produit à l'intérieur dudit support 32.

Cette installation 1 comprend donc un poste 2 de coulée et au moins un contenant de produit à couler à l'intérieur du support 32 de culture.

Dans l'exemple représenté, l'installation 1 comprend deux types de contenant appelés ci-après contenant 3 de premier type et contenant 8 de deuxième type, mais aurait pu, dans une version moins perfectionnée, comporter uniquement des contenants 3 de premier type.

Ce contenant 3 de premier type est une poche souple contenant un milieu de culture solide ou semi-solide comprenant un gélifiant, en l'occurrence ici de l'agar-agar en une teneur supérieure à 5 grammes par litre, de préférence supérieure à 10 grammes par litre, de préférence encore supérieure à 15 grammes par litre. Ce milieu de culture solide ou semi-solide contient donc au moins de l'eau et un agent gélifiant, tel que de l'agar-agar en une concentration suffisante, c'est-à-dire au moins égale à 5 grammes par litre, de préférence au moins égale à 10 grammes par litre pour former avec l'eau du milieu, un gel solide à une température généralement inférieure à 60°, tout en étant liquéfiable à ébullition, ce gel restant, après liquéfaction, en surfusion, c'est-à-dire à l'état liquide jusqu'à environ 40°C.

Un tel milieu de culture est bien connu à ceux versés dans cet art. Un tel milieu de culture est donc apte à présenter deux états : un état liquide et un état solide, en fonction des conditions de température.

De même, par poche souple, on entend une poche qui peut être déformée sous l'effet d'une pression exercée sur ladite poche à l'état liquide du milieu de culture. Cette poche 4 souple peut être réalisée au moins partiellement en matière de synthèse, en particulier en PVC ou en polypropylène.

Cette poche 4 souple est une poche stérilisable, de préférence autoclavable, à usage unique. Cette poche comprend un orifice 5 de vidange. Cette poche 4 souple peut contenir un barreau agitateur réalisée en un matériau magnétique.

Pour faciliter la vidange de la poche 4 dans l'exemple représenté, cette dernière comprend un ensemble de connexion qui comprend au moins un conduit 6 tubulaire flexible, en silicone, et une buse 7. Cet ensemble de connexion forme, avant montage sur l'orifice 5 de vidange de la poche 4, un ensemble stérile.

La buse 7 se présente sous forme d'un tube en matériau thermiquement conducteur, tel qu'un tube en métal, ouvert à chacune de ses extrémités.

Le conduit 6 tubulaire est interposé entre l'orifice 5 de vidange de la poche 4 et la buse 7, et peut être raccordé par emmanchement à l'orifice 5 de vidange et à la buse 7, de manière directe ou indirecte, c'est-à-dire avec interposition d'au moins un raccord. La poche 4 et son ensemble de connexion forment un conditionnement aisément vidangeable à l'état liquide du milieu de culture solide ou semi-solide contenu dans la poche 4.

L'orifice 5 de vidange de la poche 4 peut être, avant raccordement de l'ensemble de connexion, obturé à l'aide d'un organe d'obturation tel qu'un bouchon, amovible, éventuellement équipé d'un système de sécurité pour détecter toute ouverture non autorisée.

La poche 4 peut encore comprendre, placé à côté de l'orifice 5 de vidange, un site 35 d'injection se présentant sous forme d'une ouverture de la poche obturée à l'aide, par exemple, d'un bloc élastiquement déformable, tel qu'un élastomère, apte à être traversé par l'aiguille d'une seringue pour permettre l'adjonction d'un composant à l'intérieur de la poche.

Les contenants 8 de deuxième type sont similaires aux contenants 3 de premier type, à l'exclusion de leur contenu. Ces contenants 8 de deuxième type comprennent donc une poche 9 et un orifice de vidange 10 raccordable à une buse 7 via une conduite tubulaire 6, l'ensemble de connexion pouvant être identique, indépendamment du type de poche.

Les poches 9 constitutives des contenants de deuxième type 8 contiennent un liquide qui peut être thermosensible. Ce liquide peut constituer une infusion, un antibiotique ou toute autre solution pouvant entrer dans la composition du contenu du support de culture 32 à préparer.

Le poste 2 de coulée comprend une enceinte 11 équipée de moyens 12 de chauffage, à l'intérieur de laquelle chaque contenant 3 de premier type est apte à être stocké. Cette enceinte 11 est délimitée par une face 110 de dessus, une face 111 de dessous et des faces 112 latérales.

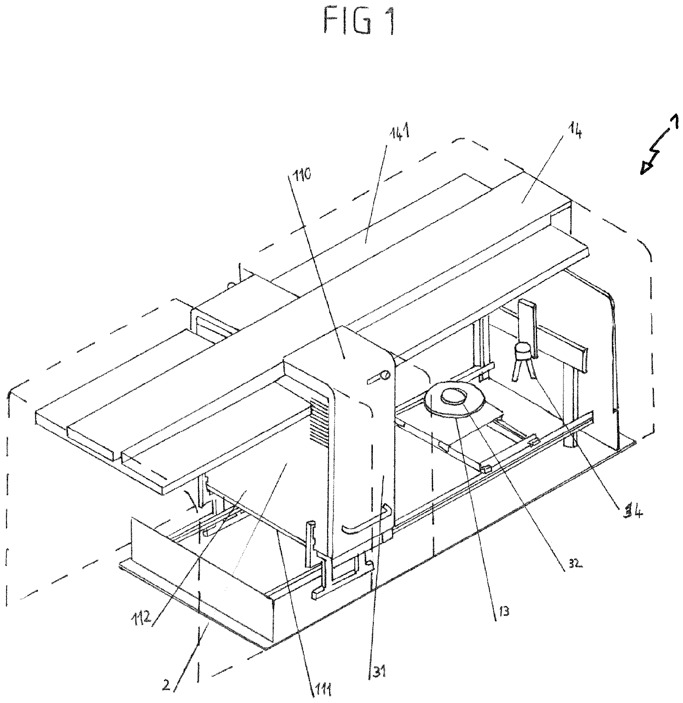

Dans l'exemple représenté, cette enceinte 11 est accolée latéralement et isolée thermiquement d'un compartiment 15 servant au stockage des contenants 8 de deuxième type. Compartiment 15 et enceinte 11 forment donc, en configuration d'utilisation de l'installation conforme à celle représentée à la figure 1, un bloc parallélipédique. Ce bloc comprend une partie fixe 30 et une partie mobile 31.

La partie mobile 31 comprend la face du dessous et l'une des faces latérales du bloc. La face du dessous du bloc forme également la face du dessous de l'enceinte 11 de stockage des contenants de premier type et accessoirement la face de dessous du compartiment 15. La paroi séparative entre compartiment et enceinte est embarquée sur la partie mobile du bloc.

Dans l'exemple représenté, la face latérale du bloc constitutive de la partie mobile 31 du bloc forme également une face latérale du compartiment 15. La partie fixe 30 du bloc comprend, à l'inverse, les autres faces latérales de l'enceinte et la face de dessus de l'enceinte. En variante, la face latérale du bloc constitutive de la partie mobile du bloc aurait pu, de manière équivalente, former une face latérale de l'enceinte 11.

La paroi de dessus du compartiment 15 est embarquée sur la partie mobile 31 du bloc, les parois latérales stationnaires de l'enceinte ménagées au niveau de la partie fixe 30 du bloc intègrent les moyens 12 de chauffage de l'enceinte 11.

Ces moyens 12 de chauffage comprennent des résistances électriques. Ces moyens 12 de chauffage sont configurés pour maintenir l'enceinte 11 de stockage des contenants de premier type à une température comprise entre 44 et 47 °C.

La face du dessus de l'enceinte, qui forme également la face du dessus du bloc, est équipée de moyens 29 d'agitation de l'air dans ladite enceinte. Ces moyens d'agitation comprennent au moins un ventilateur.

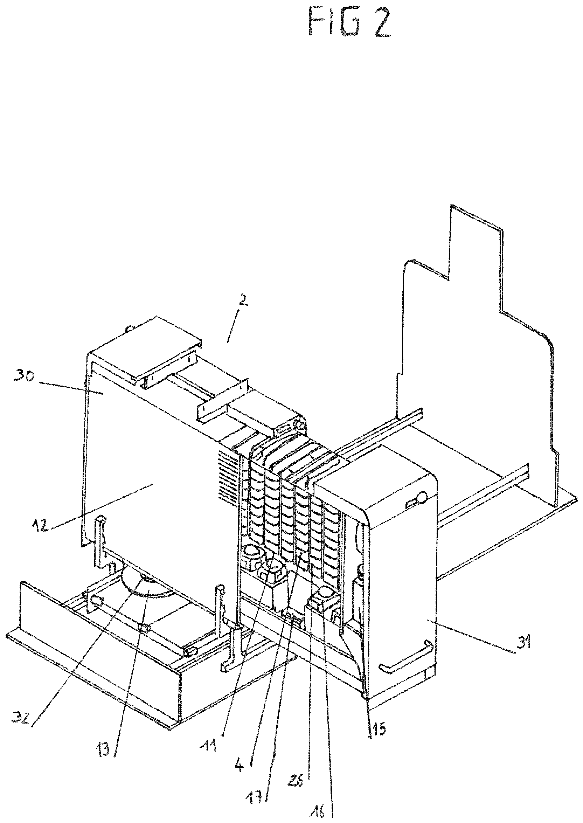

La partie mobile 31 du bloc est donc déplaçable à coulissement par rapport à la partie fixe, à la manière d'un tiroir, pour passer d'une position rapprochée de la partie fixe 30 dite configuration d'utilisation dans laquelle le bloc délimite un volume creux fermé, à une position écartée de la partie fixe 30 dite configuration de chargement et représentée à la figure 2, dans laquelle un accès à l'intérieur du bloc, et donc à l'intérieur de l'enceinte 11 et du compartiment 15 est possible, pour permettre notamment un chargement en contenant de l'enceinte et du compartiment 15.

L'installation comprend encore une surface 13 de réception du support 32 de culture à préparer et des moyens d'entraînement en déplacement relatif de l'enceinte 11 de stockage et de la surface 13 de réception.

Dans l'exemple représenté, la surface 13 de réception du support 32 de culture est formée par un plateau se déplaçant suivant une trajectoire prédéterminée, le long d'un chemin de guidage passant sous le poste de coulée. Ce chemin de guidage est délimité par deux rails parallèles le long desquels le plateau est guidé en déplacement. Des moyens moteurs ou des actionneurs tels que des vérins permettent cet entraînement en déplacement.

Dans l'exemple représenté, le plateau peut également se déplacer suivant une direction transversale à l'axe longitudinal du chemin formé par les rails. Généralement, le plateau suit une trajectoire en boucle le ramenant à un poste de chargement en support de culture, et l'installation comprend deux plateaux pour un chargement en temps masqué de chaque plateau en support de culture.

Pour chaque plateau, l'installation comprend une configuration dans laquelle ledit plateau s'étend au-dessous et à l'aplomb du poste de coulée, et en particulier de l'enceinte 11 de stockage des contenants du premier type, pour permettre un remplissage du support de culture disposé sur le plateau, au moins à partir du contenu des poches disposées dans l'enceinte 11 de stockage des contenants de premier type.

Bien que l'entraînement en déplacement de chaque plateau constitutif d'une surface 13 de réception du support 32 de culture puisse s'opérer manuellement, généralement ce déplacement s'opère automatiquement à l'aide des moteurs ou actionneurs pilotés à l'aide d'une unité de pilotage. Cette unité de pilotage est configurée pour piloter les déplacements des plateaux 13, notamment en fonction des informations disposées sur le support 32 de culture.

Des moyens de marquage de chaque support 32 de culture peuvent être prévus en au moins un emplacement déterminé de l'installation, pour permettre un marquage automatique de chaque support 32 de culture. Un tel marquage peut s'opérer par exemple par impression ou collage d'un code barre sur ledit support de culture

L'installation peut comprendre une base de données dans laquelle chaque marquage correspond à une composition de remplissage du support 32 de culture, revêtu dudit marquage.

L'installation peut encore comprendre un poste 34 d'ensemencement et des moyens d'entraînement en déplacement relatif du poste d'ensemencement et de chaque surface 13 de réception d'un support de culture pour permettre un positionnement de chaque surface 13 de réception d'une surface de culture en dessous et à l'aplomb du poste d'ensemencement.

Les moyens d'entraînement en déplacement de la surface 13 de réception du support de culture peuvent être communs aux moyens d'entraînement en déplacement de la surface 13 de réception sous le poste de coulée. Ainsi, dans une version préférentielle telle que représentée à la figure 1, l'installation peut comprendre, en sus du poste de coulée, un poste d'ensemencement et la ou chaque surface 13 de réception d'un support 32 peut être déplaçable par rapport au poste d'ensemencement et de coulée pour le passage d'un poste à un autre, suivant un ordre prédéterminé, fonction du support de culture à préparer.

Pour permettre un travail de préparation du support de culture dans des conditions stériles, le poste 2 de coulée et la surface de réception du support 32 de culture et le poste d'ensemencement lorsqu'il est présent sont logés à l'intérieur d'une salle blanche ou d'une enceinte de hotte, ladite hotte 14 de préférence à flux laminaire étant équipée de moyens 141 de production d'un flux d'air à l'intérieur de ladite enceinte de hotte comprenant au moins un ventilateur.

Pour permettre le transfert du contenu d'un contenant de premier type ou de deuxième type vers la surface 13 de réception du support 32 de culture, à l'état positionné de la surface 13 de réception du support 32 de culture au-dessous et à l'aplomb du poste de coulée, en particulier au-dessous et à l'aplomb de l'enceinte 11 de stockage, des moyens de vidange des contenants sont prévus. Ces moyens de vidange sont configurés pour permettre une vidange automatique des contenants. Ces moyens de vidange comprennent des moyens 16 de pompage. Une partie de ces moyens 16 de pompage sont disposés à l'intérieur de l'enceinte 11 de stockage pour permettre une vidange des contenants 3 de premier type. L'autre partie des moyens de pompage est ici disposée dans le compartiment 15 de stockage des contenants 8 de deuxième type.

À chaque fois, ces moyens de pompage comprennent au moins une pompe péristaltique qui peut être une pompe du commerce. Cette pompe comprend une tête de pompage formée d'éléments tournants. La pompe comprend deux mâchoires et des moyens d'entraînement en déplacement relatif des mâchoires pour le passage des mâchoires d'une position fermée à une position ouverte.

Dans la position ouverte des mâchoires, le conduit 6 tubulaire reliant l'orifice 5 de vidange de la poche 4 à la buse 7 pour le contenant le premier type ou l'orifice 10 de vidange de la poche 9 à la buse 7 pour le contenant de deuxième type est introduit dans la pompe et placé au contact de la tête rotative de la pompe, pour permettre, lors de l'entraînement en rotation de cette dernière, un écrasement du conduit générant une vidange de la poche associée.

L'installation comprend généralement autant de pompes que de contenants.

L'installation comprend encore une unité de pilotage des moyens de pompage et des moyens de saisie et d'enregistrement de données relatives au pilotage desdites pompes. L'unité de pilotage est configurée pour piloter le fonctionnement des pompes et en particulier l'entraînement en rotation des têtes de pompe en fonction des données saisies.

L'unité de pilotage comprend des moyens de traitement électroniques ou informatiques des données, tel qu'un microprocesseur associé à une mémoire de travail.

Lorsque l'expression "l'unité de pilotage est configurée pour" est utilisée, cela signifie que le microprocesseur comprend des instructions pour réaliser l'action.

Les moyens de saisie et d'enregistrement de données peuvent comprendre des moyens d'acquisition de données et/ou une interface d'entrée de données encore appelée interface homme/machine et/ou une mémoire de stockage de données prédéfinies.

Lors de la mise en place des conduits 6 dans les pompes, chaque pompe peut être appairée à une poche et à une buse. Chaque support de culture peut être revêtu d'un code d'identification correspondant à la préparation à effectuer, et les pilotages s'opèrent en fonction desdites données.

L'installation telle que représentée comprend encore un porte-buse 17 pour contenant 3 de premier type et un porte-buse 21 pour contenant 8 de deuxième type. Chaque porte-buse 17, 21 est configuré pour délimiter au moins un logement 18, 22 traversant de réception d'une buse. Dans l'exemple représenté, le porte-buse 17 pour contenant 3 de premier type est formé de deux blocs présentant chacun une série de logements traversants.

Ce porte-buse 17 pour contenant 3 de premier type est logé au moins partiellement à l'intérieur de l'enceinte 11 de stockage de contenant de premier type. Chaque buse 7 de contenant 3 de premier type débouche à l'extérieur de l'enceinte 11 de stockage des contenants 3 de premier type à l'état inséré de la buse 7 dans un logement 18 traversant du porte-buse 17 pour contenant de premier type, et chaque logement 18 traversant du porte-buse 17 apte à recevoir une buse 7 est équipé de moyens 20 de chauffage s'étendant au moins partiellement autour dudit logement 18. Les moyens 20 de chauffage sont ici formés par des résistances électriques.

Le porte-buse 17 pour contenant 3 de premier type débouche à l'extérieur de l'enceinte 11 via la face du dessous 111 de l'enceinte 11, au niveau d'une partie de la face du dessous 111 de l'enceinte 11 formée par une paroi souple réalisée ici sous forme d'une membrane 23.

En outre, chaque logement 18, 22 traversant de porte-buse 17, 21 apte à recevoir une buse 7 est monté orientable autour d'un axe dit horizontal parallèle au plan d'appui au sol de l'installation 1 ou au plan support formé par la surface 13 de réception du support 32 de culture.

Pour permettre une telle orientation, chaque série de logements 18 est disposée à l'extrémité d'un bras équipé d'un secteur circulaire. Ce secteur circulaire rotatif, denté, est en prise avec une vis sans fin. La rotation de la vis sans fin entraîne la rotation des secteurs dentés et, par suite, le pivotement du bras et de la série de logements traversants associée, pour le passage des logements associés d'une position à une autre.

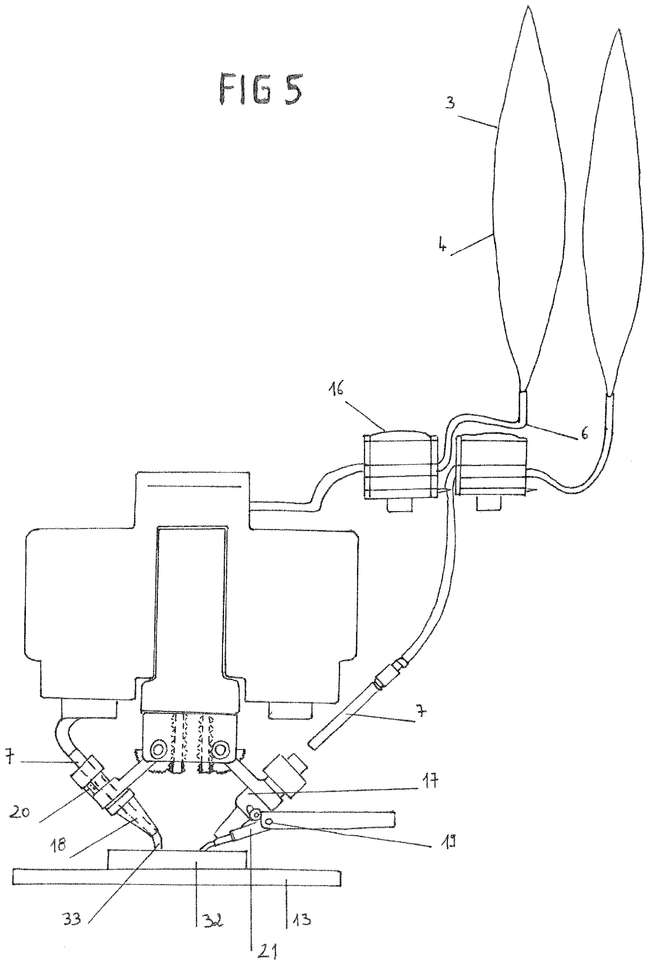

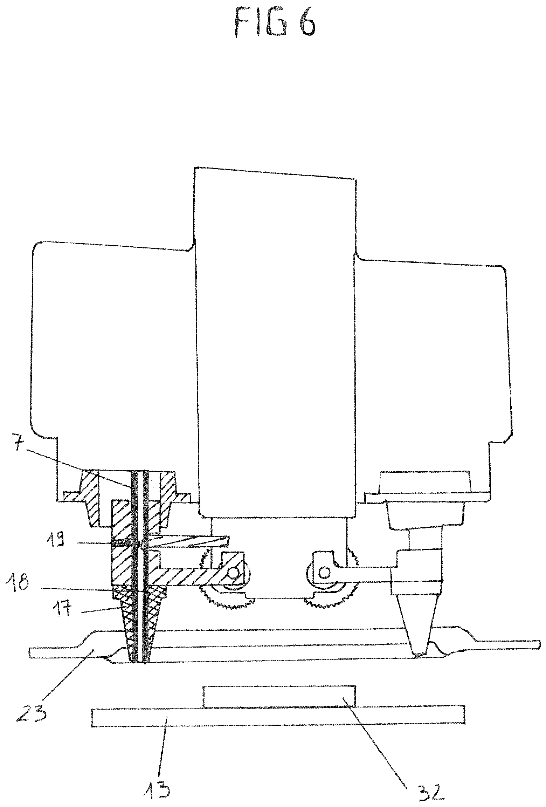

Comme l'illustre la figure 5, l'installation comprend ici deux bras dont les sections circulaires sont en prise avec une même vis sans fin, chaque bras portant une série de logements. Ainsi, les deux séries de logements traversants du porte-buse 17 pour contenant de premier type sont aptes à passer d'une position dite droite (figure 6) dans laquelle les axes longitudinaux des logements sont sensiblement parallèles entre eux et orthogonaux au plan support formé par la surface 13 de réception du support 32 de culture à une position inclinée dans laquelle chaque série de logements forme une branche d'un V.

Le poste 2 de coulée est donc équipé d'un porte-buse 17 pour contenant 3 de premier type et d'un porte-buse 21 pour contenant 8 de deuxième type et chaque porte-buse est configuré pour délimiter au moins un logement 18, 22 traversant servant à la réception d'une buse 7.

À nouveau, une unité de pilotage permet de piloter le déplacement à pivotement du porte-buses 17 en fonction du déplacement du support 32 de culture pour permettre une séquence de mouvements du support 32 de culture et du porte-buse suivant un ordre prédéfini, à savoir :
- le porte-buse 17 étant en position droite,
- le positionnement du support 32 de culture à l'aplomb du porte-buse,
- le déplacement à pivotement du porte-buses 17 de la position droite à la position inclinée,
- le déplacement suivant un mouvement vertical ascendant du support 32 de culture en vue d'un rapprochement des buses,
- le coulage de matière 33 dans le support 32 de culture,
- le déplacement suivant un mouvement vertical descendant du support 32 de culture en vue d'un écartement des buses,
- le retour du porte-buse 17 en position droite,
- l'évacuation du support de culture.

Le porte-buse 21 pour contenant 8 de deuxième type est quant à lui disposé à l'extérieur de l'enceinte 11 et du compartiment 15. Ce porte-buse 21 est porté par un bras 24 pivotant, monté mobile à pivotement autour d'un axe orthogonal au plan support formé par la surface 13 de réception du support 32 de culture pour le passage du porte-buse 21 d'une position dite active dans laquelle il s'étend au-dessous et à l'aplomb de l'enceinte 11 de stockage du ou des contenants 3 de premier type dans l'espace laissé libre entre l'enceinte 11 de stockage et le plan support formé par la surface 13 de réception du support 32 de culture à une position inactive dans laquelle il s'étend à l'extérieur dudit espace.

Ce porte-buse 21 s'étend à l'extrémité du bras et est lui-même monté orientable sur ledit bras par pivotement autour d'un axe horizontal parallèle au plan d'appui au sol de l'installation ou au plan support formé par la surface 13 de réception du support 32 de culture.

Ainsi, chaque logement 22 traversant du porte-buse 21 apte à recevoir une buse 7 est monté orientable autour d'un axe horizontal parallèle au plan d'appui au sol de l'installation ou au plan support formé par la surface 13 de réception du support 32 de culture.

Les logements 22 traversants du porte-buses 21 pour contenant de deuxième type sont montés, en position active du porte-buse 21, solidaires en déplacement des logements 18 traversants du porte-buse 17 pour contenant de premier type et sont configurés pour passer d'une position dans laquelle les axes longitudinaux des logements 22 du porte-buse 21 s'étendent parallèlement au plan support formé par la surface de réception du support de culture, à une position inclinée en direction du plan support lors du passage des logements 18 traversants du porte-buse 17 pour contenant de premier type de la position droite à la position inclinée.

Ce montage solidaire en déplacement s'opère ici grâce à la présence d'un galet monté sur le porte-buse 17 pour contenant de premier type, ce galet prenant appui sur le porte-buse 21 pour contenant de deuxième type et entraînant le porte-buse 21 en pivotement à l'encontre d'un moyen de rappel, tel qu'un ressort, rappelant le porte-buse 21 en position non inclinée.

Pour permettre un positionnement optimal d'une buse dans un logement traversant associé du porte-buse correspondant, chaque logement 18, 22 traversant est muni de moyens 19 d'indexation en position de la buse 7 à l'intérieur dudit logement. Ces moyens d'indexation sont ici formés par une bille, chargée par ressort, équipant chaque logement traversant d'un porte-buse. Chaque logement traversant associé du porte-buse peut également être équipé de moyens de détection de la présence d'une buse. Ces moyens de détection peuvent être formés par un capteur inducteur disposé à l'intérieur dudit logement. Chaque buse est quant à elle formée d'un corps tubulaire métallique muni d'une gorge annulaire périphérique externe à l'intérieur de laquelle la bille vient se loger en position insérée de la buse dans le logement traversant du porte-buse. Il en résulte un point dur qui forme un moyen de positionnement et de retenue axiale de la buse dans le porte-buse.

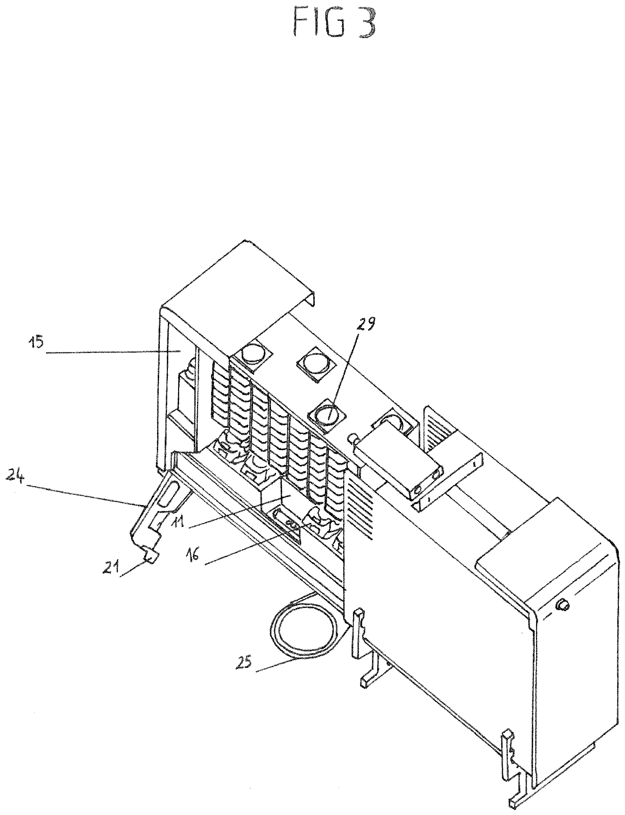

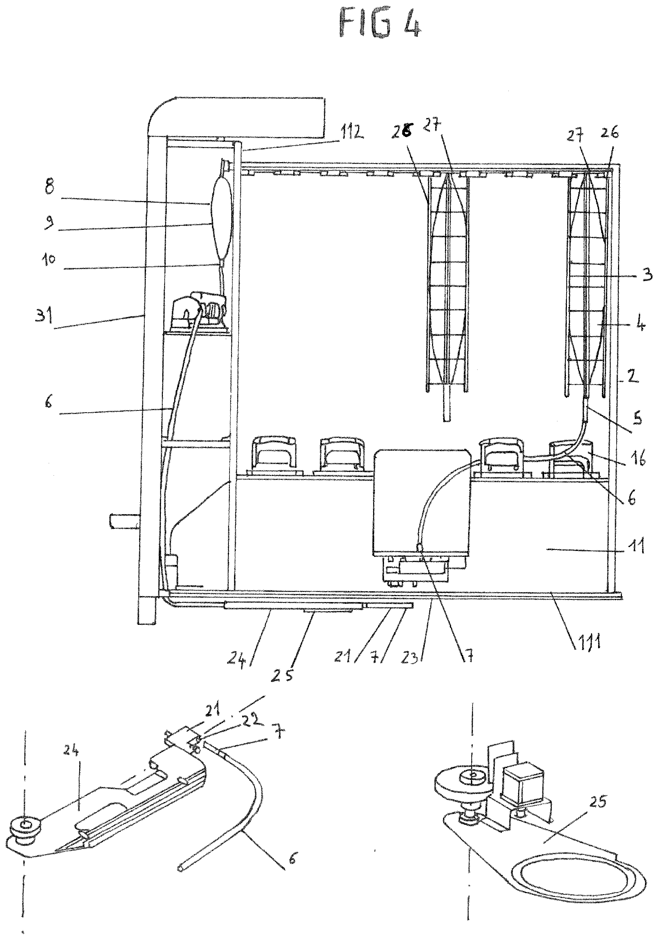

Pour parfaire le fonctionnement des buses, l'installation comprend un bras 25 pivotant équipé d'un système anti-goutte. Ce bras 25 anti-goutte, plus particulièrement visible aux figures 3 et 4 est, de manière similaire au bras 24 porte-buse, monté mobile à pivotement autour d'un axe orthogonal au plan support formé par la surface 13 de réception du support de culture pour le passage du bras anti-goutte d'une position dite active dans laquelle le système anti-goutte s'étend au-dessous et à l'aplomb de l'enceinte 11 de stockage du ou des contenants 3 de premier type dans l'espace laissé libre entre l'enceinte 11 de stockage et le plan support formé par la surface 13 de réception du support 32 de culture, à une position inactive dans laquelle il s'étend à l'extérieur dudit espace.

Le système anti-goutte est formé par une platine annulaire qui vient se positionner à l'aplomb de la sortie des buses pour empêcher toute goutte issue d'une buse de tomber sur le support de culture lorsque l'opération de coulage est achevée.

L'unité de pilotage est donc configurée pour commander un déplacement en synchronisme des porte-buses, du support 32 de culture et du bras anti-goutte tel que :
- le porte-buse pour contenant de premier type étant en position droite, le système anti-goutte du bras anti-goutte étant positionné à l'aplomb de la sortie des buses du porte-buse du contenant de premier type, le porte-buse pour contenant de deuxième type étant positionné à l'aplomb et au-dessous de l'enceinte 11 de stockage des contenants de premier type, les axes longitudinaux des logements traversants du porte-buse pour contenant de deuxième type étant positionnés parallèlement au plan support formé par la surface 13 de réception du support 32 de culture, le support de culture étant positionné à l'aplomb du porte-buse pour contenant de premier type,
- le bras anti-goutte est déplacé à pivotement pour s'étendre à l'extérieur de l'espace disposé au-dessus et à l'aplomb de l'enceinte de stockage,
- le porte-buse pour contenant de premier type est déplacé à pivotement depuis la position droite vers la position inclinée, entraînant en parallèle le déplacement du porte-buse pour contenant de deuxième type jusqu'à une position inclinée,
- le coulage est opéré,
- le porte-buse pour contenant de premier type est déplacé à pivotement depuis la position inclinée vers la position droite et le porte-buse pour contenant de deuxième type est rappelé en position parallèle au plan support,
- le bras anti-goutte est ramené dans l'espace disposé au-dessous et à l'aplomb de l'enceinte de stockage,
- le support de culture est évacué.

Pour parfaire l'installation, l'enceinte 11 de stockage de contenant de premier type est équipée de moyens de maintien des contenants de premier type à l'état suspendu à l'intérieur de ladite enceinte 11, et le compartiment 15 de stockage de contenant 8 de deuxième type est équipé de moyens de maintien des contenants de deuxième type à l'état suspendu à l'intérieur dudit compartiment.

Concernant les moyens de maintien pour chaque contenant de premier type, ces moyens de maintien comprennent un boîtier 26 grillagé à l'intérieur duquel la poche 24 constitutive du contenant 3 de premier type est apte à être logée. Ce boîtier 26 est équipé d'un organe 27 de maintien à l'état suspendu à l'intérieur de l'enceinte. Cet organe 27 de maintien est ici formé par un rail ménagé le long d'un bord du boîtier, ce rail étant apte à s'insérer à l'intérieur d'une glissière dite horizontale ménagée à l'intérieur de l'enceinte 11. Cette glissière est montée sur une structure en porte-à-faux montée sur la partie mobile du poste de coulée. Cette structure en porte-à-faux s'étend à l'horizontale au-dessus et à écartement de la face 111 de dessous de l'enceinte. Ainsi, en position écartée de la partie 31 mobile du poste de coulée de la partie 30 fixe du poste de coulée, chaque poche 10 constitutive d'un contenant 3 de premier type peut être déplacée à coulissement suivant une direction orthogonale à la direction de déplacement de la partie mobile du poste de coulée par rapport à la partie fixe, pour permettre un chargement ou un déchargement en poche de ladite partie mobile. Il en résulte une simplicité de mise en œuvre.

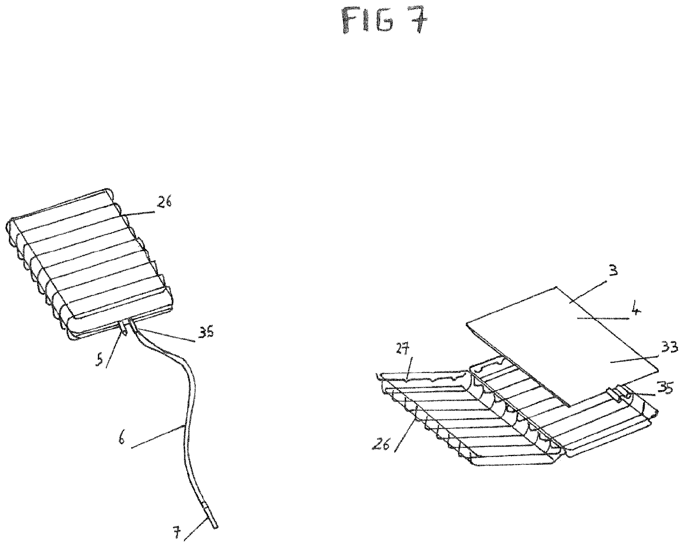

À l'état logé du contenant 3 de premier type dans le boîtier 26 et à l'état suspendu du boîtier 26 dans l'enceinte 11, l'orifice 5 de vidange du contenant 3 s'étend en partie inférieure du boîtier et fait saillie de ce dernier comme illustré à la figure 7, pour être raccordé, via le conduit 6, à la buse 7.

Ainsi, généralement, la mise en place d'un contenant de premier type dans l'enceinte 11 s'opère comme suit : la partie mobile du poste de coulée est en position écartée de la partie fixe du poste de coulée. La poche du contenant de premier type est insérée dans un boîtier grillagé. Le boîtier grillagé est suspendu à la partie mobile du poste de coulée. La buse et le conduit 6 associé du premier contenant sont respectivement insérés dans un porte-buse et une pompe. La pompe et le porte-buse sélectionnés par l'opérateur sont enregistrés généralement par l'opérateur via un écran d'affichage. Un contrôle des informations entrées par l'opérateur peut s'effectuer via un capteur de présence disposé au niveau du logement traversant de la buse et éventuellement un capteur de détection de l'ouverture de la pompe. Ainsi, chaque contenant de premier type est associé à une pompe et à un logement traversant du porte-buse.

En ce qui concerne les contenants de deuxième type, la poche du contenant est équipée d'un trou de suspension, et le compartiment comprend des crochets aptes à être enfilés dans lesdits trous. De la même manière, la buse et le conduit associé du deuxième contenant sont respectivement insérés dans un porte-buse et une pompe et leurs références sont introduites par l'opérateur dans l'installation.

Une fois les saisies de données opérées, la partie mobile du poste de coulée est ramenée en position rapprochée de la partie fixe du poste de coulée et l'installation est prête à fonctionner pour opérer des opérations de coulée en appliquant le séquençage de déplacement des porte-buses, des bras et du support de culture tel que décrit ci-dessus.

## Revendications

1. Installation (1) de préparation d'un support (32) de culture, tel qu'une boîte de Pétri, ladite installation (1) comprenant un poste (2) de coulée, au moins une surface (13), telle qu'un plateau de réception du support (32) de culture et un ou plusieurs contenants (3, 8) de produit à couler à l'intérieur dudit support (32) de culture,
**caractérisée en ce que** le ou au moins l'un des contenants de produit à couler, appelé contenant (3) de premier type, est une poche (4) souple présentant un orifice (51) de vidange et contenant un milieu (33) de culture solide ou semi-solide comprenant un gélifiant, **en ce que** le poste (2) de coulée comprend une enceinte (11) équipée de moyens (12) de chauffage aptes à chauffer l'air intérieur de l'enceinte (11), **en ce que** ladite enceinte (11) est configurée pour loger au moins deux contenants (3) de premier type, **en ce que** l'enceinte (11) de stockage du ou des contenants (3) de premier type du poste de coulée et la surface (13) de réception du support (32) de culture sont déplaçables l'une par rapport à l'autre et **en ce que** l'installation (1) comprend au moins une configuration dans laquelle la surface (13) de réception du support (32) de culture s'étend au-dessous et à l'aplomb de l'enceinte (11) de stockage du ou des contenants (3) de premier type.

2. Installation (1) selon la revendication 1,
**caractérisée en ce que** les moyens (12) de chauffage sont configurés pour maintenir l'intérieur de l'enceinte (11) de stockage du ou des contenants (3) de premier type du poste de coulée à une température comprise entre 44 et 47°C.

3. Installation (1) selon l'une des revendications 1 ou 2,
**caractérisée en ce que** le poste (2) de coulée et la surface (13) de réception du support de culture (32) sont logés à l'intérieur d'une enceinte (14) équipée de moyens (141) de production d'un flux d'air, de préférence laminaire, et de filtration dudit flux d'air ou à l'intérieur d'une salle blanche.

4. Installation (1) selon l'une des revendications 1 à 3,
**caractérisée en ce que** le poste de coulée comprend des moyens de vidange des contenants (3, 8) de produit à couler, lesdits moyens de vidange comprenant des moyens (16) de pompage des produits à couler avec au moins une partie des moyens (16) de pompage disposée à l'intérieur de l'enceinte (11) de stockage du ou des contenants (3) de premier type, cette partie des moyens (16) de pompage disposée à l'intérieur de l'enceinte (11) de stockage du ou des contenants (3) de premier type comprenant de préférence au moins une pompe telle qu'une pompe péristaltique par contenant (3) de premier type.

5. Installation (1) selon l'une des revendications 1 à 4, du type comprenant plusieurs contenants de produit à couler à savoir au moins un contenant (3) de produit à couler de premier type et au moins un contenant (8) de produit à couler dit de deuxième type, le ou au moins l'un des contenants (8) de deuxième type se présentant sous forme d'une poche (9) souple présentant un orifice (10) de vidange et contenant un liquide,
**caractérisée en ce que** le poste (2) de coulée comprend, jouxtant l'enceinte (11) de stockage du ou des contenants (3) de premier type, et isolé thermiquement de ladite enceinte (11), un compartiment (15) de stockage du ou des contenants (8) de deuxième type.

6. Installation (1) selon l'une des revendications 1 à 5, du type dont le ou chaque contenant (3, 8) de produit à couler est équipé d'un orifice (5, 10) de vidange raccordable à une buse (7),
**caractérisée en ce que** le poste (2) de coulée est équipé d'un porte-buse (17) pour contenant (3) de premier type et éventuellement d'un porte-buse (21) pour contenant (8) de deuxième type lorsque le ou les contenants (8) de deuxième type sont présents, et **en ce que** chaque porte-buse (17, 21) est configuré pour délimiter au moins un logement (18, 22) traversant servant à la réception d'une buse (7).

7. Installation (1) selon la revendication 6,
**caractérisée en ce qu'**au moins un logement (18) traversant, apte à recevoir une buse (7), du porte-buse (17) pour contenant (3) de premier type, et au moins un logement (22) traversant, apte à recevoir une buse (7), du porte-buse (21) pour contenant (8) de deuxième type présentent au moins une configuration dans laquelle ils sont positionnables à l'aplomb de la surface (13) de réception du support (32) de culture pour permettre en parallèle un coulage simultané du contenu des contenants (3) de premier type et du contenu des contenants (8) de deuxième type.

8. Installation (1) selon l'une des revendications 6 ou 7,
**caractérisée en ce que** chaque logement (18, 22) traversant est muni de moyens (19) d'indexation en position de la buse (7) à l'intérieur dudit logement.

9. Installation (1) selon l'une des revendications 6 à 8,
**caractérisée en ce que** chaque logement (18, 22) traversant de porte-buse (17, 21) apte à recevoir une buse (7) est monté orientable autour d'un axe dit horizontal parallèle au plan d'appui au sol de l'installation (1) ou au plan support formé par la surface (13) de réception du support (32) de culture.

10. Installation (1) selon l'une des revendications 6 à 9,
**caractérisée en ce que** le porte-buse (17) pour contenant (3) de premier type est logé au moins partiellement à l'intérieur de l'enceinte (11) de stockage de contenants (3) de premier type, **en ce que** chaque buse (7) de contenant (3) de premier type débouche à l'extérieur de l'enceinte (11) de stockage des contenants (3) de premier type à l'état inséré de la buse (7) dans un logement (18) traversant du porte-buse (17) pour contenants (3) de premier type, et **en ce que** chaque logement (18) traversant du porte-buse (17) apte à recevoir une buse (7) est équipé de moyens (20) de chauffage s'étendant au moins partiellement autour dudit logement (18).

11. Installation (1) selon l'une des revendications 6 à 10,
**caractérisée en ce que** l'enceinte (11) de stockage de contenants (3) de premier type comprend une face du dessus (110), une face du dessous (111) et des faces latérales (112) et **en ce que** le porte-buse (17) pour contenant (3) de premier type débouche, à l'extérieur de l'enceinte (11), via la face du dessous (111) de l'enceinte (11) au niveau d'une partie de la face du dessous (111) de l'enceinte (11) formée par une paroi souple (23), telle qu'une membrane.

12. Installation (1) selon l'une des revendications 6 à 11, prise en combinaison avec la revendication 5,
**caractérisée en ce que** le porte-buse (21) pour contenant (8) de deuxième type est porté par un bras (24) pivotant, monté mobile à pivotement autour d'un axe orthogonal au plan support formé par la surface (13) de réception du support (32) de culture pour le passage du porte-buse (21) d'une position dite active, dans laquelle il s'étend au-dessous et à l'aplomb de l'enceinte (11) de stockage du ou des contenants (3) de premier type dans l'espace laissé libre entre l'enceinte (11) de stockage et le plan support formé par la surface (13) de réception du support (32) de culture, à une position inactive dans laquelle il s'étend à l'extérieur dudit espace.

13. Installation (1) selon l'une des revendications 1 à 12,
**caractérisée en ce que** l'enceinte (11) de stockage de chaque contenant (3) de premier type est équipée de moyens de maintien des contenants (3) de premier type à l'état suspendu à l'intérieur de ladite enceinte (11), lesdits moyens de maintien comprenant, pour chaque contenant (3) de premier type à stocker, un boîtier (26) grillagé à l'intérieur duquel ledit contenant (3) de premier type est apte à être logé, ledit boîtier (26) étant équipé d'au moins un organe (27) de maintien à l'état suspendu à l'intérieur de ladite enceinte (11), ledit orifice (5) de vidange du contenant (3) de premier type s'étendant, à l'état logé du contenant (3) de premier type dans le boîtier (26) et à l'état suspendu du boîtier (26), en partie inférieure du boîtier (26).

14. Installation (1) selon l'une des revendications 1 à 13,
**caractérisée en ce que** l'enceinte (11) de stockage des contenants (3) de premier type est équipée de moyens (29) d'agitation de l'air contenu dans ladite enceinte (11).

15. Installation (1) selon l'une des revendications 1 à 14,
**caractérisée en ce que** le poste (2) de coulée se présente sous forme d'un bloc parallélépipédique comprenant une partie fixe (30) et une partie mobile (31), ladite partie mobile (31) comprenant au moins la face du dessous et l'une des faces latérales dudit bloc, cette partie mobile (31) étant déplaçable à coulissement par rapport à la partie fixe (30) à la manière d'un tiroir entre une position écartée de la partie fixe (30) et une position rapprochée de la partie fixe (30) pour permettre, en position écartée, l'accès à l'intérieur du volume formé par le bloc.

16. Installation (1) selon l'une des revendications 1 à 15,
**caractérisée en ce que** l'installation (1) comprend un poste (34) d'ensemencement, **en ce que** le poste (34) d'ensemencement et la surface (13) de réception du support (32) de culture sont déplaçables l'un par rapport à l'autre et **en ce que** l'installation (1) comprend au moins une configuration dans laquelle la surface (13) de réception du support (32) de culture s'étend au-dessous et à l'aplomb du poste (34) d'ensemencement.

## Patentansprüche

1. Anlage (1) zu Zubereitung eines Kulturträgers (32), wie z. B. einer Petrischale, wobei die Anlage (1) eine Gießstation (2), mindestens eine Fläche (13) wie z. B. eine Aufnahmeebene des Kulturträgers (32) und ein oder mehrere Behältnisse (3, 8) von Produkt, das in das Innere des Kulturträgers (32) gegossen werden soll, umfasst,
**dadurch gekennzeichnet, dass** das oder mindestens eines der Behältnisse von Produkt, das gegossen werden soll, bezeichnet als Behältnis (3) der ersten Art, ein flexibler Beutel (4) ist, der ein Ablaufloch (51) aufweist und ein festes oder halbfestes Kulturmedium (33) enthält, umfassend ein Geliermittel, dadurch, dass die Gießstation (2) einen Behälter (11) umfasst, der mit Heizmitteln (12) ausgestattet ist, die ausgelegt sind, um das Innere (11) des Behälters zu heizen, dadurch, dass der Behälter (11) konfiguriert ist, um mindestens zwei Behältnisse (3) der ersten Art unterzubringen, dadurch, dass der Speicherbehälter (11) des oder der Behältnisse (3) der ersten Art der Gießstation und die Fläche (13) zur Aufnahme des Kulturträgers (32) mit Bezug aufeinander verschiebbar sind, und dadurch, dass die Anlage (1) mindestens eine Konfiguration umfasst, in der sich die Fläche (13) zur Aufnahme des Kulturträgers (32) über den und senkrecht zum Speicherbehälter (11) des oder der Behältnisse (3) der ersten Art erstreckt.

2. Anlage (1) nach Anspruch 1,
**dadurch gekennzeichnet, dass** die Heizmittel (12) konfiguriert sind, um das Innere des Speicherbehälters (11) des oder der Behältnisse (3) der ersten Art der Gießstation auf einer Temperatur zu halten, die zwischen 44 und 47 °C liegt.

3. Anlage (1) nach einem der Ansprüche 1 oder 2,
**dadurch gekennzeichnet, dass** die Gießstation (2) und die Fläche (13) zur Aufnahme des Kulturträgers (32) im Inneren eines Behälters (14), der mit Mitteln (141) zur Erzeugung eines vorzugsweise laminaren Luftstroms und zur Filterung des Luftstroms ausgestattet ist, oder im Inneren eines Reinraums untergebracht sind.

4. Anlage (1) nach einem der Ansprüche 1 bis 3,
**dadurch gekennzeichnet, dass** die Gießstation Mittel zum Ablaufen der Behältnisse (3, 8) von Produkt, das gegossen werden soll, umfasst, wobei die Mittel zum Ablaufen Mittel zum Pumpen (16) der Produkte, die gegossen werden sollen, umfassen, wobei mindestens ein Teil der Mittel zum Pumpen (16) im Inneren des Speicherbehälters (11) des oder der Behältnisse (3) der ersten Art angeordnet sind, wobei dieser Teil der Mittel zum Pumpen (16), der im Inneren des Speicherbehälters (11) des oder der Behältnisse(s) (3) der ersten Art angeordnet sind, vorzugsweise mindestens eine Pumpe umfassen, wie z. B. eine peristaltische Pumpe pro Behältnis (3) der ersten Art.

5. Anlage (1) nach einem der Ansprüche 1 bis 4 der Art, umfassend mehrere Behältnisse von Produkt, das gegossen werden soll, d. h. mindestens ein Behältnis (3) von Produkt der ersten Art, das gegossen werden soll, und mindestens ein Behältnis (8) von Produkt, das gegossen werden soll, bezeichnet als der zweiten Art, wobei das oder mindestens eines der Behältnisse (8) der zweiten Art die Form eines flexiblen Beutels (9) aufweisen, der ein Ablaufloch (10) aufweist und eine Flüssigkeit enthält,
**dadurch gekennzeichnet, dass** die Gießstation (2) angrenzend an den Speicherbehälter (11) des oder der Behältnisse (3) der ersten Art, und thermisch von dem Behälter (11) isoliert, ein Speicherabteil (15) des oder der Behältnisse (8) der zweiten Art umfasst.

6. Anlage (1) nach einem der Ansprüche 1 bis 5 der Art, bei der das oder jedes Behältnis (3, 8) des Produkts, das gegossen werden soll, mit einem Ablaufloch (5, 10) ausgestattet ist, das mit einer Düse (7) verbunden werden kann,
**dadurch gekennzeichnet, dass** die Gießstation (2) mit einem Düsenträger (17) für ein Behältnis (3) der ersten Art und eventuell mit einem Düsenträger (21) für ein Behältnis (8) der zweiten Art ausgestattet ist, wenn das oder die Behältnisse (8) der zweiten Art vorhanden sind, und dadurch, dass jeder Düsenträger (17, 21) konfiguriert ist, um mindestens eine durchgehende Unterbringung (18, 22) zu begrenzen, die zur Aufnahme einer Düse (7) dient.

7. Anlage (1) nach Anspruch 6,
**dadurch gekennzeichnet, dass** mindestens eine durchgehende Unterbringung (18), die ausgelegt ist, um eine Düse (7) des Düsenträgers (17) für ein Behältnis (3) der ersten Art aufzunehmen, und mindestens eine durchgehende Unterbringung (22), die ausgelegt ist, um eine Düse (7) des Düsenträgers (21) für ein Behältnis (8) der zweiten Art aufzunehmen, mindestens eine Konfiguration aufweisen, in der sie senkrecht zur Fläche (13) zur Aufnahme des Kulturträgers (32) positioniert werden können, um parallel ein gleichzeitiges Ablaufen des Inhalte der Behältnisse (3) der ersten Art und des Inhalts der Behältnisse (8) der zweiten Art zu ermöglichen.

8. Anlage (1) nach einem der Ansprüche 6 oder 7,
**dadurch gekennzeichnet, dass** jede durchgehende Unterbringung (18, 22) mit Anzeigemitteln (19) in der Position der Düse (7) im Inneren der Unterbringung versehen ist.

9. Anlage (1) nach einem der Ansprüche 6 bis 8,
**dadurch gekennzeichnet, dass** jede durchgehende Unterbringung (18, 22) der Trägerdüse (17, 21), die ausgelegt ist, um eine Düse (7) aufzunehmen, orientierbar um eine als horizontal bezeichnete Achse, parallel zur Auflageebene auf dem Boden der Anlage (1) oder zur Stützebene, gebildet von der Aufnahmefläche des Kulturträgers (32), montiert ist.

10. Anlage (1) nach einem der Ansprüche 6 bis 9,
**dadurch gekennzeichnet, dass** der Düsenträger (17) für das Behältnis (3) der ersten Art mindestens teilweise im Inneren des Speicherbehälters (11) zur Speicherung von Behältnissen (3) der ersten Art untergebracht ist, dadurch, dass jede Düse (7) eines Behältnisses (3) der ersten Art im Inneren des Speicherbehälters (11) der Behältnisse (3) der ersten Art, im eingeführten Zustand der Düse (7) in eine durchgehende Unterbringung (18) des Düsenträgers (17) für Behältnisse (3) der ersten Art, mündet, und dadurch, dass jede durchgehende Unterbringung (18) der Trägerdüse (17), die ausgelegt ist, um eine Düse (7) aufzunehmen, mit Heizmitteln (20) ausgestattet ist, die sich mindestens teilweise um die Unterbringung (18) erstrecken.

11. Anlage (1) nach einem der Ansprüche 6 bis 10,
**dadurch gekennzeichnet, dass** der Speicherbehälter (11) der Behältnisse (3) der ersten Art eine obere Seite (110), eine untere Seite (111) und laterale Seiten (112) umfasst, und dadurch, dass der Düsenträger (17) für ein Behältnis (3) der ersten Art an der Außenseite des Behälters (11) über die untere Seite (111) des Behälters (11) auf der Ebene eines Teils der unteren Seite (111) des Behälters (11), gebildet von einer flexiblen Wand (23), wie z. B. einer Membran, mündet.

12. Anlage (1) nach einem der Ansprüche 6 bis 11 in Kombination mit Anspruch 5, **dadurch gekennzeichnet, dass** der Düsenträger (21) für ein Behältnis (8) der zweiten Art von einem Schwenkarm (24) getragen wird, der beweglich schwenkend um eine zur Trägerebene orthogonale Achse montiert ist, die von der Fläche (13) zur Aufnahme des Kulturträgers (32) gebildet ist, für den Durchgang des Düsenträgers (21) von einer als aktiv bezeichneten Position, in der er sich unter dem und senkrecht zum Speicherbehälter (11) des oder der Behältnisse(s) (3) der ersten Art im Raum erstreckt, der zwischen dem Speicherbehälter (11) und der Stützebene, die von der Fläche (13) zur Aufnahme des Kulturträgers (32) gebildet ist, frei gelassen ist, in eine inaktive Position , in der er sich auf die Außenseite des Raums erstreckt.

13. Anlage (1) nach einem der Ansprüche 1 bis 12,
**dadurch gekennzeichnet, dass** der Speicherbehälter (11) jedes Behältnisses (3) der ersten Art mit Mitteln zur Beibehaltung der Behältnisse (3) der ersten Art im aufgehängten Zustand im Inneren des Behälters (11) ausgestattet ist, wobei die Mittel zur Beibehaltung für jedes Behältnis (3) der ersten Art, das gespeichert werden soll, ein vergittertes Gehäuse (26) umfassen, in dessen Innerem das Behältnis (3) der ersten Art ausgelegt ist, um aufgenommen zu werden, wobei das Gehäuse (26) mit mindestens einem Organ (27) ausgestattet ist, um im Inneren des Behälters (11) im aufgehängten Zustand gehalten zu werden, wobei sich das Ablaufloch (5) des Behältnisses (3) der ersten Art im Zustand, in dem das Behältnis (3) der ersten Art im Gehäuse (26) untergebracht ist, und im aufgehängten Zustand des Gehäuses (26) im unteren Teil des Gehäuses (26) erstreckt.

14. Anlage (1) nach einem der Ansprüche 1 bis 13,
**dadurch gekennzeichnet, dass** der Speicherbehälter (11) der Behältnisse (3) der ersten Art mit Mitteln (29) zur Bewegung der Luft, die im dem Behälter (11) enthalten ist, ausgestattet ist.

15. Anlage (1) nach einem der Ansprüche 1 bis 14,
**dadurch gekennzeichnet, dass** die Gießstation (2) die Form eines parallelepipeden Blocks aufweist, umfassend einen festen Teil (30) und einen beweglichen Teil (31), wobei der bewegliche Teil (31) mindestens die untere Seite und eine der lateralen Seiten des Blocks umfasst, wobei dieser bewegliche Teil (31) durch Verschiebung mit Bezug auf den festen Teil (30) auf die Art eines Schubladens zwischen einer Position entfernt vom festen Teil (30) und eine Position angenähert an den festen Teil (30) verschoben werden kann, um in der entfernten Position den Zugriff auf das Innere des Volumens, gebildet vom Block, zu ermöglichen.

16. Anlage (1) nach einem der Ansprüche 1 bis 15,
**dadurch gekennzeichnet, dass** die Anlage (1) eine Aussaatstation (34) umfasst, dadurch, dass die Aussaatstation (34) und die Fläche (13) zur Aufnahme des Kulturträgers (32) mit Bezug aufeinander verschiebbar sind, und dadurch, dass die Anlage (1) mindestens eine Konfiguration umfasst, in der sich die Fläche (13) zur Aufnahme des Kulturträgers (32) unter der und senkrecht zur Aussaatstation (34) erstreckt.

## Claims

1. A facility (1) for preparing a culture support (32), such as a petri dish, said facility (1) comprising a pouring station (2), at least one surface (13), such as a plate for receiving the culture support (32) and one or several containers (3, 8) for a product to be poured inside said culture support (32),
**characterized in that** the or at least one of the containers of product to be poured, called container (3) of the first type, is a flexible pouch (4) having an emptying orifice (51) and containing a solid or semi-solid culture medium (33) comprising a gelling agent, **in that** the pouring station (2) comprises an enclosure (11) equipped with heating means (12) able to heat the air inside the enclosure (11), **in that** said enclosure (11) is configured to house at least two containers (3) of the first type, **in that** the storage enclosure (11) of the container(s) (3) of the first type of the pouring station and the surface (13) for receiving the culture support (32) are movable relative to one another and **in that** the installation (1) comprises at least one configuration in which the surface (13) for receiving the culture support (32) extends below and in line with the storage enclosure (11) of the container(s) (3) of the first type.

2. The facility (1) according to claim 1,
**characterized in that** the heating means (12) are configured to keep the inside of the storage enclosure (11) of the container(s) (3) of the first type of the pouring station at a temperature between 44 and 47°C.

3. The facility (1) according to one of claims 1 or 2,
**characterized in that** the pouring station (2) and the surface (13) for receiving the culture support (32) are housed inside an enclosure (14) equipped with means (141) for producing a flow of air, preferably laminar, and filtering said flow of air or inside a clean room.

4. The facility (1) according to one of claims 1 to 3,
**characterized in that** the pouring station comprises means for emptying the containers (3, 8) of product to be poured, said emptying means comprising means (16) for pumping products to be poured with at least part of the pumping means (16) arranged inside the storage enclosure (11) of the container(s) (3) of the first type, this part of the pumping means (16) arranged inside the storage enclosure (11) of the container(s) (3) of the first type preferably comprising at least one pump such as a peristaltic pump per container (3) of the first type.

5. The facility (1) according to one of claims 1 to 4, of the type comprising several containers of product to be poured, namely at least one container (3) of product to be poured of the first type and at least one container (8) of product to be poured which is said to be of the second type, the or at least one of the containers (8) of the second type assuming the form of a flexible pouch (9) having an emptying orifice (10) and containing a liquid,
**characterized in that** the pouring station (2) comprises, adjoining the storage enclosure (11) of the container(s) (3) of the first type, and thermally insulated from said enclosure (11), a storage compartment (15) of the container(s) (8) of the second type.

6. The facility (1) according to one of claims 1 to 5, of the type whereof the or each container (3, 8) of product to be poured is equipped with an emptying orifice (5, 10) connectable to a nozzle (7), **characterized in that** the pouring station (2) is equipped with a nozzle carrier (17) for a container (3) of the first type and optionally a nozzle carrier (21) for a container (8) of the second type when the container(s) (8) of the second type are present, and **in that** each nozzle carrier (17, 21) is configured to delimit at least one through housing (18, 22) used to receive a nozzle (7).

7. The facility (1) according to claim 6,
**characterized in that** at least one through housing (18), able to receive a nozzle (7), of the nozzle carrier (17) for a container (3) of the first type, and at least one through housing (22), able to receive a nozzle (7), of the nozzle carrier (21) for a container (8) of the second type have at least one configuration in which they can be positioned in line with the receiving surface (13) of the culture support (32) to allow, in parallel, simultaneous pouring of the content of the containers (3) of the first type and the content of the containers (8) of the second type.

8. The facility (1) according to one of claims 6 or 7,
**characterized in that** each through housing (18, 22) is provided with means (19) for indexing the position of the nozzle (7) inside said housing.

9. The facility (1) according to one of claims 6 to 8,
**characterized in that** each nozzle carrier (17, 21) through housing (18, 22) able to receive a nozzle (7) is mounted such that it can be oriented about a so-called horizontal axis parallel to the bearing plane on the ground of the facility (1) or to the support plane formed by the surface (13) for receiving the culture support (32).

10. The facility (1) according to one of claims 6 to 9,
**characterized in that** the nozzle carrier (17) for a container (3) of the first type is housed at least partially inside the storage enclosure (11) for containers (3) of the first type, **in that** each nozzle (7) of a container (3) of the first type emerges outside the storage enclosure (11) for the containers (3) of the first type in the state of the nozzle (7) inserted into a through housing (18) of the nozzle carrier (17) for containers (3) of the first type, and **in that** each through housing (18) of the nozzle holder (17) able to receive a nozzle (7) is equipped with heating means (20) extending at least partially around said housing (18).

11. The facility (1) according to one of claims 6 to 10,
**characterized in that** the storage enclosure (11) for containers (3) of the first type comprises a top face (110), a bottom face (111) and side faces (112) and **in that** the nozzle carrier (17) for a container (3) of the first type emerges, outside the enclosure (11), via the bottom face (111) of the enclosure (11) at the part of the bottom face (111) of the enclosure (11) formed by a flexible wall (23), such as a membrane.

12. The facility (1) according to one of claims 6 to 11, combined with claim 5,
**characterized in that** the nozzle holder (21) for a container (8) of the second type is supported by a pivoting arm (24), mounted pivoting about an axis orthogonal to the support plane formed by the surface (13) for receiving the culture support (32) for the passage of the nozzle carrier (21) from a so-called active position, in which it extends below and in line with the storage enclosure (11) for the container(s) (3) of the first type in the space left free between the storage enclosure (11) and the support plane formed by the surface (13) for receiving the culture support (32), to an inactive position in which it extends outside said space.

13. The facility (1) according to one of claims 1 to 12,
**characterized in that** the storage enclosure (11) of each container (3) of the first type is equipped with means for maintaining the containers (3) of the first type in the suspended state inside said enclosure (11), said maintaining means comprising, for each container (3) of the first type to be stored, a wire housing (26) inside which said container (3) of the first type is able to be housed, said housing (26) being equipped with at least one maintaining member (27) for maintaining in the suspended state inside said enclosure (11), said emptying orifice (5) of the container (3) of the first type extending, in the state of the container (3) of the first type housed in the housing (26) and in the suspended state of the housing (26), in the lower part of the housing (26).

14. The facility (1) according to one of claims 1 to 13,
**characterized in that** the storage facility (11) of the containers (3) of the first type is equipped with means (29) for agitating the air contained in said enclosure (11).

15. The facility (1) according to one of claims 1 to 14,
**characterized in that** the pouring station (2) assumes the form of a parallelepipedal block comprising a stationary part (30) and a mobile part (31), said mobile part (31) comprising at least the bottom face and one of the side faces of said block, this mobile part (31) being slidingly movable relative to the stationary part (30) like a drawer between a position separated from the stationary part (30) and a position close to the stationary part (30) to allow, in the separated position, access to the inside of the volume formed by the block.

16. The facility (1) according to one of claims 1 to 15,
**characterized in that** the facility (1) comprises a seeding station (34), **in that** the seeding station (34) and the surface (13) for receiving the culture support (32) are movable relative to one another and **in that** the facility (1) comprises at least one configuration in which the surface (13) for receiving the culture support (32) extends below and in line with the seeding station (34).
